(19) Europäisches Patentamt
European Patent Office
Office européen des brevets

(11) Publication number: **0 450 660 B1**

(12) # EUROPEAN PATENT SPECIFICATION

(45) Date of publication of patent specification: **09.03.94 Bulletin 94/10**

(51) Int. Cl.$^5$: **C07C 275/30,** A61K 31/17

(21) Application number: **91107475.5**

(22) Date of filing: **20.11.89**

(54) **Anti-atherosclerotic diaryl compound.**

(30) Priority: **21.11.88 GB 8827152**

(43) Date of publication of application: **09.10.91 Bulletin 91/41**

(60) Publication number of the earlier application in accordance with Art. 76 EPC: **0 370 740**

(45) Publication of the grant of the patent: **09.03.94 Bulletin 94/10**

(84) Designated Contracting States: **AT BE CH DE ES FR GB GR IT LI LU NL SE**

(56) References cited:
**EP-A- 0 283 742**
**EP-A- 0 297 610**
**US-A- 4 387 106**
**US-A- 4 603 145**

(73) Proprietor: **THE WELLCOME FOUNDATION LIMITED**
**Unicorn House 160 Euston Road**
**London NW1 2BP (GB)**

(72) Inventor: **Jackson, William Paul**
**The Wellcome Research Laboratories, Langley Court**
**Beckenham, Kent BR3 3BS (GB)**
Inventor: **Harris, Clifford John**
**The Wellcome Research Laboratories, Langley Court**
**Beckenham, Kent BR3 3BS (GB)**
Inventor: **Arrowsmith, Richard James**
**The Wellcome Research Laboratories, Langley Court**
**Beckenham, Kent BR3 3BS (GB)**
Inventor: **Dann, John Gordon**
**The Wellcome Research Laboratories, Langley Court**
**Beckenham, Kent BR3 3BS (GB)**
Inventor: **O'Connor, Kevin Julian**
**The Wellcome Research Laboratories, Langley Court**
**Beckenham, Kent BR3 3BS (GB)**
Inventor: **Booth, Robert Frederick Geoffrey**
**The Wellcome Research Laboratories, Langley Court**
**Beckenham, Kent BR3 3BS (GB)**

(74) Representative: **Rollins, Anthony John et al**
**Group Patents & Agreements The Wellcome Research Laboratories Langley Court**
**Beckenham Kent BR3 3BS (GB)**

EP 0 450 660 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

ANTI-ATHEROSCLEROTIC DIARYL COMPOUND

The present invention is concerned with a new diaryl compound, processes for its preparation, compositions containing it and their use in medicine, particularly in the prophylaxis or treatment of atherosclerosis.

Intracellular cholesterol ester metabolism in the arterial wall is handled by a variety of enzymes including acyl coenzyme A : cholesterol acyl transferase (ACAT), cholesteryl ester hydrolase (CEH), acid cholesterol hydrolase and cholesteral esterase. Of these, ACAT and cholesteryl ester hydrolase appear to control the steady state concentration of cholesterol ester in the arterial wall :

$$\text{Cholesterol} \underset{\text{CEH}}{\overset{\text{ACAT}}{\rightleftharpoons}} \text{Cholesterol ester}$$

ACAT may also play a key role in the gastrointestinal absorption of cholesterol on the basis that (a) more than 90% of the cholesterol which appears in the lymph is esterified, (b) substantial ACAT activity has been observed in the intestinal mucosal cells of several animal species, (c) the site of greatest intestinal ACAT activity is the jejunum where the majority of cholesterol absorption occurs, (d) ACAT activity in the jejunum parallels increases in dietary cholesterol and (e) ACAT activity is significantly enhanced in animals having experimental atherosclerosis and in atherosclerotic human tissue and cell cultures.

Compounds having ACAT-inhibitory activity are known from US Patent 4,603,145 and European Patent Application 0297610. USP '145 describes novel diarylalkanamides useful as pharmaceutical agents for ameliorating atherosclerosis by inhibiting the formation and development of atherosclerotic lesions in the arterial wall of mammals. EPA '610 describes substituted urea, thiourea, carbamate and thiocarbamate derivatives which are potent inhibitors of ACAT and are thus useful agents for inhibiting the intestinal absorption of cholesterol.

The following references describe bridged diaryl compounds, some of which are reported to have therapeutic utility in the treatment of conditions which are not associated with ACAT, for example, depression and hypertension:

DE-A-2340874
EP-A-0017484
WO-A-84/02704
Chem. Abs. 98:215869z
Chem. Abs. 83:9602u
J. Org. Chem. 46, 4416 (1981)
Chem. Abs. 97:38593m
JCS Chem. Comm. 1979, 499
Chem. Ber. 105, 1634 (1972)
Chem. Ber. 96, 765 (1963)
Coll. Czech. Chem. Comm. 33, 1880 (1968)
J. Med. Chem. 13 35 (1970)

We have discovered a novel class of bridged diaryl compounds which exhibit ACAT inhibitory activity and which are particularly useful as hypolipidaemics as well as for decreasing the steady state concentration of cholesterol and cholesterol ester in the arterial wall and thereby retarding the build-up of atherosclerotic lesions. All but one of these compounds form the subject matter of European Patent Specification 0370740. The remaining compound, which is the most preferred in the class, is the subject of the present divisional application.

According to the present invention, therefore, there is provided the compound of formula (I)

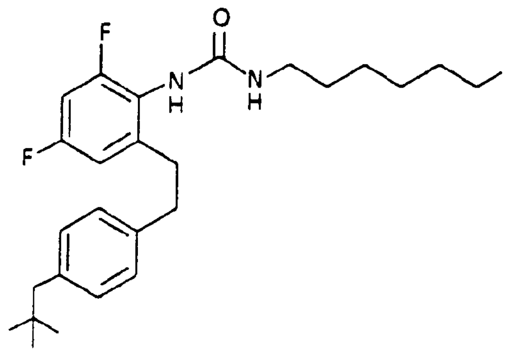

(I)

known as 1-[2,4-difluoro-6-{2-[4-(2,2-dimethylpropyl)phenyl]ethyl} phenyl]-3-heptylurea; and salts and solvates thereof.

Salts of the compound of formula (I) suitable for use in medicine are those which are physiologically acceptable. However, non-physiologically acceptable salts are within the scope of the present invention for use as intermediates in the preparation of the compound of the invention and its physiologically acceptable salts and solvates.

According to further aspects of the invention, there are also provided :

(a) the compound of formula (I) and pharmaceutically acceptable salts and solvates thereof for use in therapy;

(b) pharmaceutical formulations comprising the compound of formula (I) and/or one of its pharmaceutically acceptable salts or solvates and at least one pharmaceutical carrier or excipient;

(c) the use of the compound of formula (I) or of a pharmaceutically acceptable salt or solvate thereof in the manufacture of a medicament for the prophylaxis or treatment of a clinical condition for which an ACAT inhibitor is indicated;

(d) a method for the prophylaxis or treatment of a clinical condition in a mammal, such as a human, for which an ACAT inhibitor is indicated which comprises the administration of a therapeutically effective amount of the compound of formula (I) or of a pharmaceutically acceptable salt or solvate thereof to said mammal; and

(e) processes for the preparation of the compound of formula (I) (including salts and solvates thereof).

With regard to aspects (a), (c) and (d), the ability of the compound of formula (I) to inhibit ACAT activity renders it useful as a hypolipidaemic and in reducing the steady state concentration of cholesterol and cholesterol ester in the arterial wall, thereby retarding the build-up of atherosclerotic lesions and providing application for this compound in the prophylaxis or treatment of atherosclerosis.

Hereinafter all references to "the compound of formula (I)" include its salts and solvates.

The amount of the compound of formula (I) which is required to achieve the desired biological effect will, of course, depend on a number of factors, for example, the use for whith it is intended, the mode of administration, and the clinical condition of the recipient. In general, a daily dose is expected to lie in the range of from 1ng to 100mg, typically from 50ng to 50mg, per day per kilogram bodyweight, for example, 500ng-5mg/kg/day. An intravenous dose may, for example, be in the range of from 10ng to 1 mg/kg which may conveniently be administered as an infusion of from 0.1ng to 50g per kilogram per minute. Infusion fluids suitable for this purpose may contain, for example, from 0.01ng to 100g, typically from 0.1ng to 100g, per millilitre. Unit doses, for example, may contain from 100ng to 100mg of the active compound; for example, ampoules for injection may contain from 100ng to 1mg and orally administrable unit dose formulations, such as tablets or capsules, may contain, for example, from 0.001 to 50mg, typically from 0.02 to 20mg. In the case of physiologically acceptable salts, the weights indicated above refer to the weight of the diaryl ion derived from the salt.

For the prophylaxis or treatment of the conditions referred to above, the compound of formula (I) may be used as the compound per se, but is preferably presented with an acceptable carrier as a pharmaceutical formulation. The carrier must, of course, be acceptable in the sense of being compatible with the other ingredients of the formulation and not be deleterious to the recipient thereof. The carrier may be a solid or a liquid, or both, and is preferably formulated with the compound as a unit dose formulation, for example, a tablet, which may contain from 0.05% to 95% by weight of the compound. Other pharmacologically active substances may also be present in the formulations of the present invention. One or more forms of the compound of formula (I) may be incorporated in the formulations of the invention, which may be prepared by any of the well known techniques of pharmacy consisting essentially of admixture of the components.

The formulations include those suitable for oral, rectal, topical, buccal (e.g. sub-lingual) and parenteral (e.g.

subcutaneous, intramuscular, intradermal, or intravenous) administration, although the most suitable route in any given case will depend on the nature and severity of the condition being treated and on the nature of the particular form of the compound of formula (I) which is being used.

Formulations suitable for oral administration may be presented in discrete units, such as capsules, cachets, lozenges, or tablets, each containing a predetermined amount of the compound of formula (I) or of a physiologically acceptable salt thereof; as a powder or granules; as a solution or suspension in an aqueous or non-aqueous liquid; or as an oil-in-water or water-in-oil emulsion. Such formulations may be prepared by any suitable method of pharmacy which includes the step of bringing into association the compound and the carrier (which may constitute one or more accessory ingredients). In general, the formulations are prepared by uniformly and intimately admixing the compound with a liquid or finely divided solid carrier, or both, and then, if necessary, shaping the product. For example, a tablet may be prepared by compressing or moulding a powder or granules of the compound, optionally with one or more accessory ingredients. Compressed tablets may be prepared by compressing, in a suitable machine, the compound in a free-flowing form, such as a powder or granules optionally mixed with a binder, lubricant, inert diluent and/or surface-active/dispersing agent(s). Moulded tablets may be made by moulding, in a suitable machine, the powdered compound moistened with an inert liquid diluent.

Formulations suitable for buccal (sub-lingual) administrations include lozenges comprising the compound of formula (I), or a physiologically acceptable salt thereof, in a flavoured base, usually sucrose and acacia or tragacanth; and pastilles comprising the compound in an inert base such as gelatin and glycerin or sucrose and acacia.

Formulations of the present invention suitable for parenteral administration conveniently comprise sterile aqueous preparations of the compound of formula (I), or of a physiologically acceptable salt thereof, which preparations are preferably isotonic with the blood of the recipient. The preparations are preferably administered intravenously, although administration may also be effected by means of subcutaneous, intramuscular, or intradermal injection. Such preparations may conveniently be prepared by admixing the compound with water and rendering the resulting solution sterile and isotonic with the blood. Injectable compositions according to the invention will generally contain from 0.1 to 5% w/w of the compound of formula (I).

Formulations suitable for rectal administration are preferably presented as unit dose suppositories. These may be prepared by admixing the compound of formula (I), or a physiologically acceptable salt thereof, with one or more conventional solid carriers, for example, cocoa butter, and then shaping the resulting mixture. Formulations suitable for topical application to the skin preferably take the form of an ointment, cream, lotion, paste, gel, spray, aerosol, or oil. Carriers which may be used include vaseline, lanoline, polyethylene glycols, alcohols and combinations of two or more therof. The active compound is generally present at a concentration of from 0.1 to 15% w/w of the composition, for example, from 0.5 to 2%.

The compound of formula (I) may be prepared in any conventional manner, for example, by the process described below. According to this aspect of the invention, the compound of formula (I) may be prepared by reacting a compound of formula (II)

$$C_7H_{15}\text{-}P \qquad (II)$$

wherein P is as defined below, with a compound of formula (III)

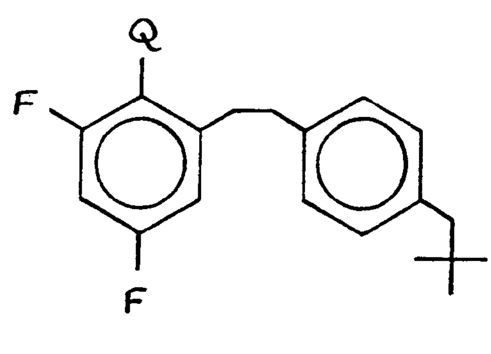

(III)

wherein Q is a nucleophilic group, for example, amino, capable of reacting with the group P, for example, isocyanate, in compound (II) to give the compound of formula (I). The reaction is typically carried out in a non-polar solvent, such as THF, in the presence of a suitable base, for example, DMAP.

Compounds of formula (III) may be prepared by reacting a compound of formula (IV)

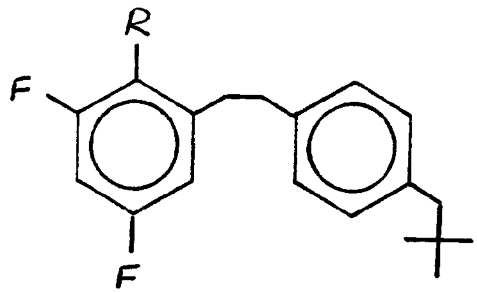

(IV)

wherein R is a group capable of being converted to the group Q of compound (III), with a suitable reagent or reagents and under such conditions as to effect conversion of R to Q, for example, converting a nitro group to an amino group by catalytic hydrogenation.

Compounds of formula (IV) may be prepared by selectively reducing the carbonyl group of a compound of formula (V)

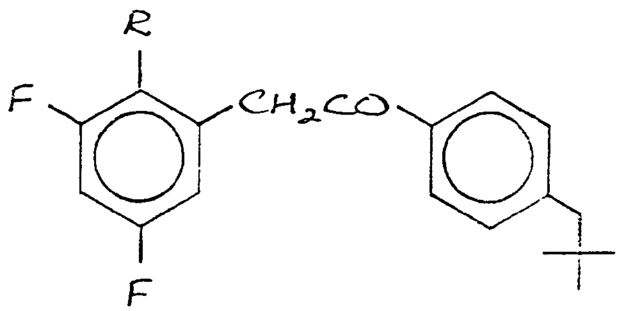

(V)

wherein R is as hereinbefore defined, using, for example, triethylsilane in the presence of trifluoroacetic acid.

Compounds of formula (V) may be prepared by reacting a compound of formula (VI)

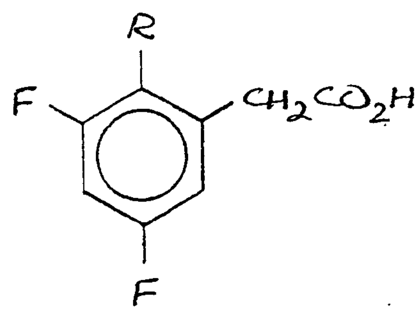

(VI)

wherein R is as hereinbefore defined, with a compound of formula (VII)

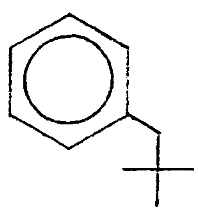

(VII)

using, for example, Friedel-Crafts acylation conditions.

Compounds of formula (VI) may be prepared by reacting a compound of formula (VIII)

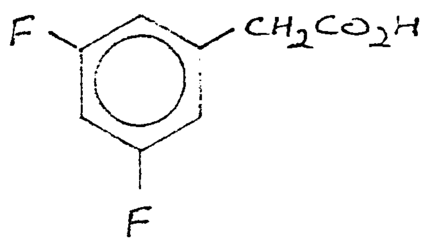

(VIII)

with a suitable reagent or reagents and under such conditions as to substitute the group R of compound (VI) in the o-position, for example, in the case where R is nitro, by selective nitration using c.$H_2SO_4$/c.$HNO_3$ at low temperature.

Compounds of formulae (II) and (VII) are commercially available or may be prepared by methods well known to those skilled in the art or readily available from the chemical literature.

Optional conversion of the compound of formula (I) to a corresponding salt may be effected by reaction with the appropriate acid or base.

For a better understanding of the invention, the following Examples are given by way of illustration.

Synthetic Example

Preparation of 1-[2,4-difluoro-6-{2-[4-(2,2-dimethylpropyl)phenyl]ethyl}phenyl]-3-heptylurea

(a) 2-Nitro-3,5-difluorophenyl acetic acid

A solution of 3,5-difluorophenylacetic acid (20g, Fluorochem) in c.$H_2SO_4$ (150ml) was stirred at -15°C and c.$HNO_3$ (75ml) added dropwise over 1 hour at a temperature of from -5 to -10°C. The mixture was stirred for a further 1/2 hour at -10°C and then poured into ice-water (1.0 1). The latter was extracted with ether (x3) and the combined extracts washed with water (x2), dried over $MgSO_4$, and evaporated under reduced pressure to give a solid which was crystallised and then recrystallised from ether/hexane (1:4 v/v) to give 20.8g of the desired product.

Analysis: Calcd C 44.24; H 2.30; N 6.45
Found C 44.18; H 2.38; N 6.24

200MHz $^1$H NMR consistent with proposed structure.

(b) 2,4-Difluoro-6-[4-(2,2-dimethylpropyl)benzoylmethyl]-1-nitrobenzene

A suspension of the product from step (a) (10g) in neopentyl benzene (40ml, Fluka) was stirred at room temperature and oxalyl chloride (8.8g) was added dropwise over 5 minutes, then DMF (5-10 drops). The mixture was stirred overnight at room temperature, then evaporated under reduced pressure to give an orange/yellow solution to which $AlCl_3$ (7.4g) was added portionwise over 15 minutes. The resulting mixture was stirred for 1/2 hour at room temperature, followed by 4 hours at 60°C, then poured into ice/c.HCl (300g/50ml). The latter was extracted with ethyl acetate (x3) and the combined extracts washed with 2N HCl, 2N aqu. NaOH (x2), 2N HCl, and water (x2), dried over $MgSO_4$/charcoal, and evaporated under reduced pressure to give a brown oil which was flash chromatographed through a silica column using ether/hexane (3:7 v/v). The eluate was evaporated under reduced pressure and the residue crystallised from ether/hexane (1:3 v/v) to give 6.3g of the desired product.

Analysis: Calcd C 65.72; H 5.54; N 3.99
Found C 65.71; H 5.48; N 4.03

200MHz $^1$H NMR consistent with proposed structure.

(c) 2,4-Difluoro-6-{2-[4-(2,2-dimethylpropyl)phenyl]ethyl}-1-nitrobenzene

A solution of the product from step (b) (11.1g) in trifluoroacetic acid (16ml) was stirred at room temperature and triethylsilane (12.5ml) added. The mixture was stirred overnight at room temperature, followed by 3 hours at 50°C during which further portions of triethylsilane (3ml each) were added after 1.5 hours and 2.5 hours, then poured into water (250ml). The latter was extracted with ether (x2) and the combined extracts washed with water (x2), dried over $MgSO_4$, and evaporated under reduced pressure to give an oil which was flash chromatographed through a silica column using ether/hexane (1:4 v/v). The eluate was evaporated under reduced pressure to give 11.0g of the desired product.

Analysis: Calcd 68.47; H 6.31; N 4.20
Found 68.75; H 6.75; N 4.24

200MHz $^1$H NMR consistent with proposed structure.

(d) 2,4-Difluoro-6-{2-[4-(2,2-dimethylpropyl)phenyl]ethyl}aniline

10% Pd/C (600mg) was added under nitrogen to a stirred solution of the product from step (c) (10.9g)

in ethanol (150ml) and the mixture hydrogenated at room temperature at a pressure of 1 atm. $H_2$ for 3.5 hours (uptake 2300ml). The mixture was filtered, the residue washed with ethanol, and the filtrate evaporated under reduced pressure to give an oil which crystallised on standing to give 8.0g of the desired product.

Analysis: Calcd C 75.25; H 7.59; N 4.62
Found C 75.61; H 8.01; N 4.62

200 MHz $^1$H NMR consistent with proposed structure.

(e) 1-[2,4-Difluoro-6-{2-[4-(2,2-dimethylpronyl)phenyl]ethyl)}phenyl]-3-heptylurea

A solution of the product from step (d) (4.5g) in THF (50ml) was stirred at room temperature and heptyl isocyanate (5.5g containing 36 mole % benzene, prepared by the method described in Organic Syntheses, Collective Volume 3, p.846) and N,N-dimethylaminopyridine (750mg) were added. The mixture was stirred at room temperature for ca 60 hours, then evaporated under reduced pressure to give an oil which was flash chromatographed through a silica column using ether/hexane (2:1 v/v). The eluate was evaporated under reduced pressure and the residue crystallised from hexane and triturated with methanol to give 4.5g of material. The latter was flash chromatographed through a silica column using ether/hexane (2:1 v/v) and then freeze-dried from dioxan to give 5.3g of the desired product, mp 105-107°C.

Analysis: Calcd C 72.97; H 8.56; N 6.31
Found C 72.16; H 8.45; N 6.17

200MHz $^1$H NMR consistent with proposed structure.

## Pharmaceutical Formulation Examples

In the following Examples, the "active ingredient" may be the compound of formula (I) as hereinbefore defined.

### (i) Tablet formulations

The following formulations A, B and C may be prepared by wet granulation of ingredients (a) to (c), (a) to (d) and (a) to (c) respectively with a solution of povidone, followed by addition of the magnesium stearate and compression.

### Formulation A

| | | mg/tablet | mg/tablet |
|---|---|---|---|
| (a) | Active ingredient | 250 | 250 |
| (b) | Lactose B.P. | 210 | 26 |
| (c) | Sodium Starch Glycollate | 20 | 12 |
| (d) | Povidone B.P. | 15 | 9 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose 150 | 150 | - |
| (c) | Avicel PH 101 | 60 | 26 |
| (d) | Sodium Starch Glycollate | 20 | 12 |
| (e) | Povidone B.P. | 15 | 9 |
| (f) | Magnesium Stearate | 5 | 3 |
| | | 500 | 300 |

Formulation C

| | | mg/tablet |
|---|---|---|
| (a) | Active ingredient | 100 |
| (b) | Lactose | 200 |
| (c) | Starch | 50 |
| (d) | Povidone | 5 |
| (e) | Magnesium Stearate | 4 |
| | | 359 |

The following formulations D and E may be prepared by direct compression of the admixed ingredients. The lactose used in formulation E is of the direct compression type.

Formulation D

| | mg/capsule |
|---|---|
| Active ingredient | 250 |
| Magnesium Stearate | 4 |
| Pregelatinised Starch NF15 | 146 |
| | 400 |

Formulation E

| | mg/capsule |
|---|---|
| Active ingredient | 250 |
| Magnesium Stearate | 5 |
| Lactose | 145 |
| Avicel | 100 |
| | 500 |

Formulation F (Controlled release formulation)

| | | mg/tablet |
|---|---|---|
| (a) | Active ingredient | 500 |
| (b) | Hydroxypropylmethylcellulos (Methocel K4M Premium) | 112 |
| (c) | Lactose B.P. | 53 |
| (d) | Povidone B.P.C. | 28 |
| (e) | Magnesium Stearate | 7 |
| | | 700 |

The formulation may be prepared by wet granulation of ingredients (a) to (c) with a solution of povidone, followed by addition of the magnesium stearate and compression.

(ii) Capsule formulations

Formulation A

Capsules may be prepared by admixing the ingredients of Formulation D above and filling two-part hard gelatin capsules with the resulting mixture. Formulation B (infra) may be prepared in a similar manner.

Formulation B

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Formulation C

| | | mg/capsule |
|---|---|---|
| (a) | Active ingredient | 250 |
| (b) | Macrogel 4000 BP | 350 |
| | | 600 |

Capsules may be prepared by melting the Macrogel 4000 BP, dispersing the active ingredient in the melt, and filling two-part hard gelatin capsules therewith.

Formulation D

| | mg/capsule |
|---|---|
| Active ingredient | 250 |
| Lecithin | 100 |
| Arachis Oil | 100 |
| | 450 |

Capsules may be prepared by dispersing the active ingredient in the lecithin and arachis oil and filling soft, elastic gelatin capsules with the dispersion.

Formulation E (Controlled release capsule)

| | | mg/capsule |
|---|---|---|
| (a) | Active ingredient | 250 |
| (b) | Microcrystalline Cellulose | 125 |
| (c) | Lactose BP | 125 |
| (d) | Ethyl Cellulose | 13 |
| | | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

(iii) Intravenous injection formulation

Active ingredient 0.200g
Sterile, pyrogend-free phosphate buffer (pH 9.0) to 10 ml

The active ingredient is dissolved in most of the phosphate buffer at 35-40°C, then made up to volume and filtered through a sterile micropore filter into sterile 10 ml glass vials (Type 1) which are sealed with sterile closures and overseals.

(iv) Intramuscular injection formulation

Active ingredient 0.20 g
Benzyl Alcohol 0.10 g
Glycofurol 75 1.45 g
Water for Injection q.s. to 3.00 ml

The active ingredient is dissolved in the glycofurol. The benzyl alcohol is then added and dissolved, and water added to 3 ml. The mixture is then filtered through a sterile micropore filter and sealed in sterile 3 ml glass vials (Type 1).

(v) Syrup formulation

Active ingredient 0.2500 g
Sorbitol Solution 1.5000 g
Glycerol 1.0000 g
Sodium Benzoate 0.0050 g
Flavour 0.0125 ml
Purified Water q.s. to 5.0 ml

The sodium benzoate is dissolved in a portion of the purified water and the sorbitol solution added. The

active ingredient is added and dissolved. The resulting solution is mixed with the glycerol and then made up to the required volume with the purified water.

(vi) Suppository formulation

| | mg/suppository |
|---|---|
| Active ingredient (63μm)* | 250 |
| Hard Fat, BP (Witepsol H15 - Dynamit Nobel) | 1770 |
| | 2020 |

* The active ingredient is used as a powder wherein at least 90% of the particles are of 63μm diameter or less.

One-fifth of the Witepsol H15 is melted in a steam-jacketed pan at 45°C maximum. The active ingredient is sifted through a 200μm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250μm stainless steel screen and, with continuous stirring, allowed to cool to 40°C. At a temperature of 38-40°C, 2.02g aliquots of the mixture are filled into suitable plastic moulds and the suppositories allowed to cool to room temperature.

(vii) Pessary formulation

| | mg/pessary |
|---|---|
| Active ingredient (63μm) | 250 |
| Anhydrous Dextrose | 380 |
| Potato Starch | 363 |
| Magnesium Stearate | 7 |
| | 1000 |

The above ingredients are mixed directly and pessaries prepared by compression of the resulting mixture.

Biological assay

In vitro inhibition of ACAT

The in vitro esterification of cholesterol in the presence of ACAT and the test compound was assayed radiometrically using [$^{14}$C]oleoyl CoA as substrate:

[$^{14}$C]oleoyl CoA + cholesterol $\rightarrow$ [$^{14}$C]oleoyl cholesterol + CoASH

The enzyme is membrane-associated in vivo. Microsomal protein is therefore used as the source of both ACAT and cholesterol. The compound of the invention was tested against enzyme derived from human embryo 407 intestinal epithelial cell line.

[$^{14}$C]Oleoyl CoA was incubated with microsomal protein at 37°C, pH 7.0, in the presence of various concentrations of the test compound. After 4 minutes, the reaction was stopped by the addition of ice-cold chloroform/methanol containing a known amount of [$^{3}$H]oleoyl cholesterol to compensate for the loss of any [$^{14}$C] product. A known volume of the resulting lower phase, which contains lipidic material from the reaction, was dried, redissolved in hexane containing unlabelled oleoyl cholesterol (TLC marker) and run on a quantitative

TLC plate (silica gel). The oleoyl cholesterol spot was visualised (iodine vapour), removed from the TLC plate and its radioactivity measured by scintillation counting.

A plot of ACAT inhibitory activity vs concentration was prepared for the test compound and the corresponding $IC_{50}$ determined. The compound of the invention was found to significantly inhibit ACAT with an $IC_{50}$ for ACAT inhibition of 0.022μM.

Toxicity

The cytotoxicity of the compound of the invention was investigated in vitro by studying its effects on the metabolic competence of isolated rat liver cells. No effect on gluconeogenesis was observed at concentrations of up to 100μM. A 15% decrease in ATP levels was observed after 90 minutes at a concentration of 100μM.

## Claims

**Claims for the following Contracting States : DE, GB, FR, IT, NL, SE, LI, CH, BE, AT, LU**

1. The compound of formula (I)

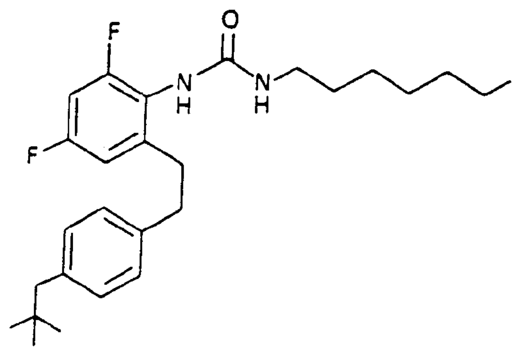

(I)

known as 1-[2,4-difluoro-6-(2-[4-(2,2-dimethylpropyl)phenyl]ethyl)-phenyl]-3-heptylurea.

2. A salt or solvate of the compound of formula (I) claimed in claim 1.

3. A pharmaceutical formulation comprising the compound of formula (I) claimed in claim 1 or a physiologically acceptable salt or solvate thereof as claimed in Claim 2, a pharmaceutically acceptable carrier or excipient and, optionally, one or more other pharmacologically active agents.

4. A formulation as claimed in claim 3 which is in the form of a tablet or capsule.

5. A process for the preparation of the compound of formula (I) claimed in claim 1 which comprises reacting a compound of formula (II)

$$C_7H_{15}\text{-}P \quad \text{(II)}$$

with a compound of formula (III)

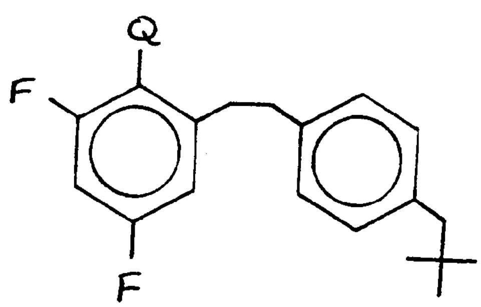

(III)

either P in the compound of formula (II) or Q in the compound of formula (III) being a nucleophilic group capable of reacting with Q in the compound of formula (III) or, P in the compound of formula (II) respectively to give the compound of formula (I).

6. A process as claimed in claim 5, wherein P in the compound of formula (II) is an isocyanate group and Q in the compound of formula (III) is an amino group.

7. A process as claimed in claim 5 or 6, wherein the compound of formula (I) so formed is converted to a corresponding salt or solvate.

8. The compound of formula (I) as claimed in claim 1, or a physiologically acceptable salt or solvate thereof, for use in therapy.

9. The compound of formula (I) as claimed in claim 1, or a physiologically acceptable salt or solvate thereof, for use in the prophylaxis or treatment of a clinical condition for which an ACAT inhibitor is indicated.

10. The compound of formula (I) as claimed in claim 1, or a physiologically acceptable salt or solvate thereof, for use in the prophylaxis or treatment of atherosclerosis.

11. Use of the compound of formula (I) as claimed in claim 1, or of a physiologically acceptable salt or solvate thereof, in the manufacture of a medicament for the prophylaxis or treatment of a clinical condition for which an ACAT inhibitor is indicated.

12. Use of the compound of formula (I) claimed in claim 1, or of a physiologically acceptable salt or solvate thereof, in the manufacture of a medicament for the prophylaxis or treatment of atherosclerosis.

**Claims for the following Contracting States : ES, GR**

1. A process for the preparation of the compound of formula (I)

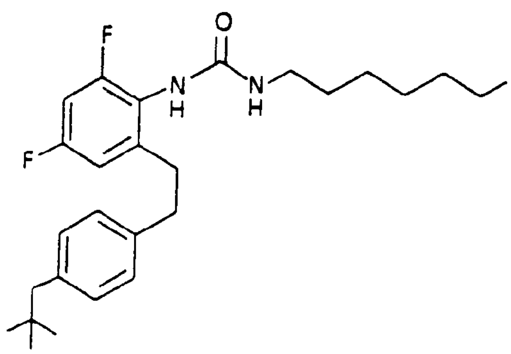

(I)

which process comprises reacting a compound of formula (II)

$C_7H_{15}$-P (II)

with a compound of formula (III)

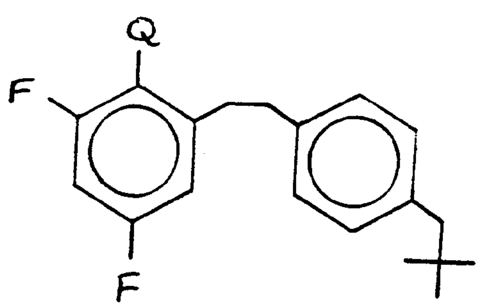

(III)

either P in the compound of formula (II) or Q in the compound of formula (III) being a nucleophilic group

capable of reacting with Q in the compound of formula (III) or, P in the compound of formula (II) respectively to give the compound of formula (I).

2. A process as claimed in claim 1, wherein P in the compound of formula (II) is an isocyanate group and Q in the compound of formula (III) is an amino group.

3. A process as claimed in claim 1 or 2, wherein the compound of formula (I) so formed is converted to a corresponding salt or solvate.

4. A method of preparing a pharmaceutical formulation which comprises
   (a) preparing the compound of formula (I) or a physiologically acceptable salt or solvate thereof by a process as claimed in any of claims 1 to 3; and
   (b) admixing the product from step (a) with a pharmaceutically acceptable carrier or excipient and, optionally, one or more other pharmacologically active agents.

5. A method as claimed in claim 4 which comprises an additional step (c) wherein the admixture from step (b) is formed into a tablet or capsule.

## Patentansprüche

### Patentansprüche für folgende Vertragsstaaten : DE, GB, FR, IT, NL, SE, LI, CH, BE, AT, LU

1. Verbindung der Formel (I)

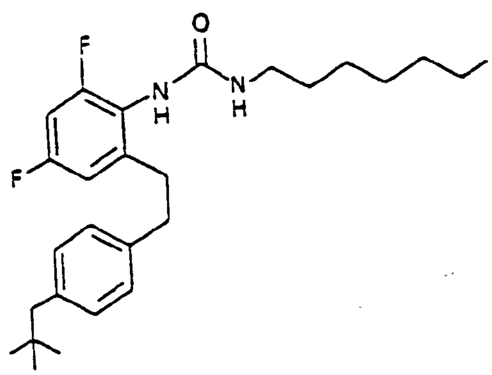

(I)

bekannt als 1-[2,4-Difluoro-6-(2-[4-(2,2-dimethylpropyl)phenyl]ethyl)phenyl]-3-heptylharnstoff.

2. Salz oder Solvat der Verbindung der Formel (I) nach Anspruch 1.

3. Pharmazeutische Formulierung, umfassend die Verbindung der Formel (I) nach Anspruch 1 oder ein physiologisch akzeptables Salz oder Solvat davon nach Anspurch 2, einen pharmazeutisch akzeptablen Träger oder Exzipienten und gegebenenfalls einen oder mehrere pharmakologisch aktive Mittel.

4. Formulierung nach Anspruch 3, die in der Form einer Tablette oder Kapsel ist.

5. Verfahren zur Herstellung der Verbindung der Formel (I) nach Anspruch 1, umfassend die Reaktion einer Verbindung der Formel (II)

$$C_7H_{15}\text{-}P \qquad (II)$$

mit einer Verbindung der Formel (III)

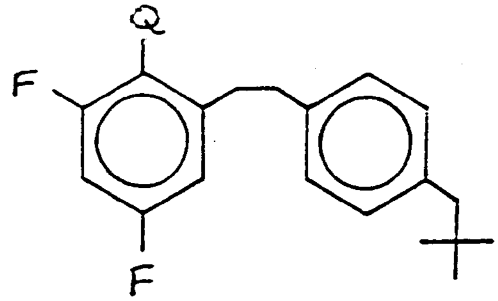

(III)

wobei entweder P in der Verbindung der Formel (II) oder Q in der Verbindung der Formel (III) eine nukleophile Gruppe ist, die in der Lage ist, mit Q in der Verbindung der Formel (III) bzw. P in der Verbindung der Formel (II) zu reagieren, unter Erhalt der Verbindung der Formel (I).

6. Verfahren nach Anspruch 5, worin P in der Verbindung der Formel (II) eine Isocyanatgruppe ist, und worin Q in der Verbindung der Formel (III) eine Aminogruppe ist.

7. Verfahren nach Anspruch 5 oder 6, worin die so gebildete Verbindung der Formel (I) in ein korrespondierendes Salz oder Solvat umgewandelt wird.

8. Verbindung der Formel (I) nach Anspruch 1 oder ein physiologisch akzeptables Salz oder Solvat davon zur Verwendung in der Therapie.

9. Verbindung der Formel (I) nach Anspruch 1 oder ein physiologisch akzeptables Salz oder Solvat davon zur Verwendung bei der Prophylaxe oder Behandlung eines klinischen Zustandes, für den ein ACAT-Inhibitor angezeigt ist.

10. Verbindung der Formel (I) nach Anspruch 1 oder ein physiologisch akzeptables Salz oder Solvat davon zur Verwendung in der Prophylaxe oder Behandlung von Arteriosklerose.

11. Verwendung der Verbindung der Formel (I) nach Anspruch 1 oder eines phyiologisch akzeptablen Salzes oder Solvates davon, bei der Herstellung eines Medikamentes für die Prophylaxe oder Behandlung eines klinischen Zustandes, für den ein ACAT-Inhibitor angezeigt ist.

12. Verwendung der Verbindung der Formel (I) nach Anspruch 1 oder eines physiologisch akzeptablen Salzes oder Solvates davon, bei der Herstellung eines Medikamentes für die Prophylaxe oder Behandlung von Arteriosklerose.

**Patentansprüche für folgende Vertragsstaaten : ES, GR**

1. 1. Verfahren zur Herstellung der Verbindung der Formel (I)

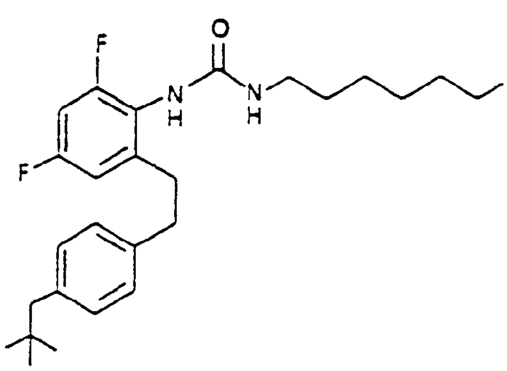

(I)

wobei das Verfahren die Reaktion einer Verbindung der Formel (II)

$C_7H_{15}$-P (II)

mit einer Verbindung der Formel (III)

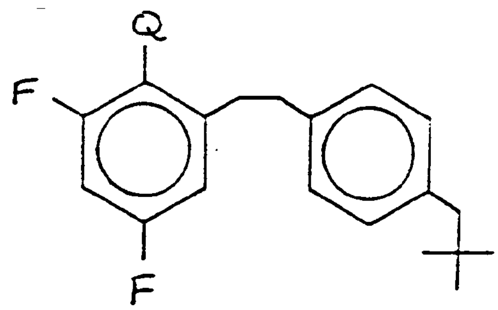

(III)

umfaßt, worin entweder P in der Verbindung der Formel (II) oder Q in der Verbinaung der Formel (III) eine nukleophile Gruppe ist, die in der Lage ist, mit Q in der Verbindung der Formel (III) bzw. P in der Verbindung der Formel (II) zu reagieren, unter Erhalt der Verbindung der Formel (I).

2. Verfahren nach Anspruch 1, worin P in der Verbindung der Formel (II) eine Isocyanatgruppe ist, und worin Q in der Verbindung der Formel (III) eine Aminogruppe ist.

3. Verfahren nach Anspruch 1 oder 2, worin die so gebildete Verbindung der Formel (I) in ein korrespondierendes Salz oder Solvat umgewandelt wird.

4. Verfahren zur Herstellung einer pharmazeutischen Formulierung, umfassend
   (a) Herstellung der Verbindung der Formel (I) oder eines physiologisch akzeptablen Salzes oder Solvates davon durch ein Verfahren nach einem der Ansprüche 1 bis 3; und
   (b) Vermischen des Produktes von Schritt (a) mit einem pharmazeutisch akzeptablen Träger oder Exzipienten und gegebenenfalls einem oder mehreren anderen pharmakologisch aktiven Mitteln.

5. Verfahren nach Anspruch 4, umfassend einen zusätzlichen Schritt (c), worin die Zumischung von Schritt (b) in eine Tablette oder Kapsel gebildet wird.

## Revendications

### Revendications pour les Etats contractants suivants : DE, GB, FR, IT, NL, SE, LI, CH, BE, AT, LU

1. Composé de formule (I) :

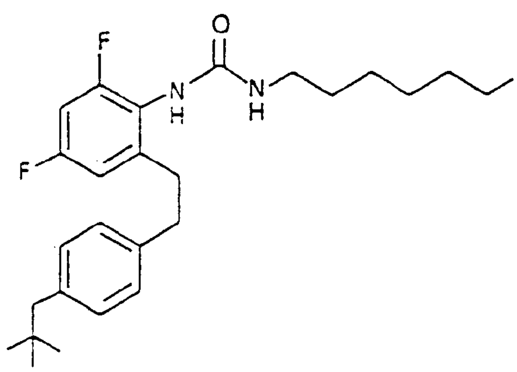

(I)

connu comme la 1-[2,4-difluoro-6-{2-[4-(2,2-diméthylpropyl)phényl]éthyl}phényl]-3-heptylurée.

2. Sel ou solvate du composé de formule (I) suivant la revendication 1.

3. Composition pharmaceutique comprenant le composé de formule (I) suivant la revendication 1, ou un sel ou solvate physiologiquement acceptable de celui-ci suivant la revendication 2, un excipient ou diluant pharmaceutiquement acceptable et facultativement un ou plusieurs autres agents pharmacologiquement

actifs.

4. Composition suivant la revendication 3, qui se trouve sous la forme d'un comprimé ou d'une capsule.

5. Procédé de préparation du composé de formule (I) suivant la revendication 1, qui comprend la réaction d'un composé de formule (II) :

$$C_7H_{15}\text{-}P \qquad (II)$$

avec un composé de formule (III) :

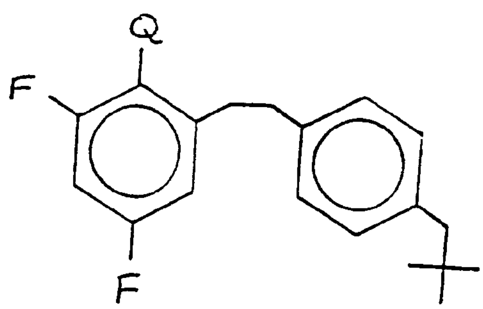

(III)

P dans le composé de formule (II) ou Q dans le composé de formule (III) étant un radical nucléophile capable de réagir avec Q dans le composé de formule (III) ou, P dans le composé de formule (II), respectivement, pour donner le composé de formule (I).

6. Procédé suivant la revendication 5, où P dans le composé de formule (II) est un radical isocyanate et Q dans le composé de formule (III) est un radical amino.

7. Procédé suivant la revendication 5 ou 6, où le composé de formule (I) ainsi obtenu est converti en un sel ou solvate correspondant.

8. Composé de formule (I) suivant la revendication 1, ou un sel ou solvate physiologiquement acceptable de celui-ci, à utiliser en thérapeutique.

9. Composé de formule (I) suivant la revendication 1, ou un sel ou solvate physiologiquement acceptable de celui-ci, à utiliser dans la prophylaxie ou le traitement d'un état clinique pour lequel un inhibiteur de l'ACAT est indiqué.

10. Composé de formule (I) suivant la revendication 1, ou un sel ou solvate physiologiquement acceptable de celui-ci, à utiliser dans la prophylaxie ou le traitement de l'athérosclérose.

11. Utilisation d'un composé de formule (I) suivant la revendication 1, ou d'un sel ou solvate physiologiquement acceptable de celui-ci, dans la fabrication d'un médicament pour la prophylaxie ou le traitement d'un état clinique pour lequel un inhibiteur de l'ACAT est indiqué.

12. Utilisation d'un composé de formule (I) suivant la revendication 1, ou d'un sel ou solvate physiologiquement acceptable de celui-ci dans la fabrication d'un médicament pour la prophylaxie ou le traitement de l'athérosclérose.

**Revendications pour les Etats contractants suivants : ES, GR**

1. Procédé pour préparer un composé de formule (I) :

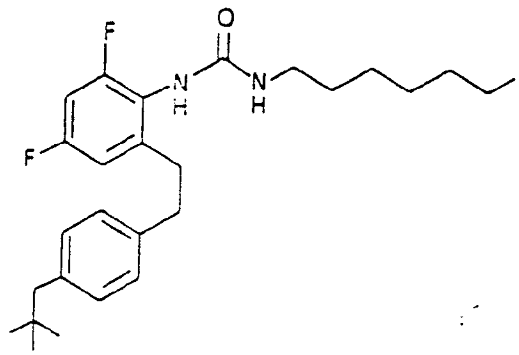

(I)

lequel procédé comprend la réaction d'un composé de formule (II) :

$C_7H_{15}$-P (II)

avec un composé de formule (III) :

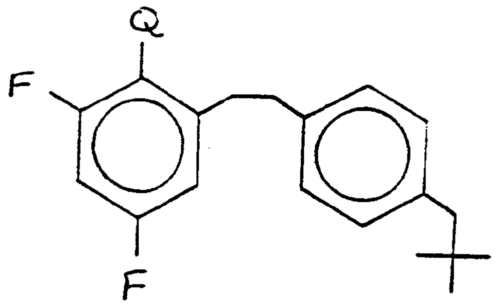

(III)

P dans le composé de formule (II) ou Q dans le composé de formule (III) étant un radical nucléophile capable de réagir avec Q dans le composé de formule (III) ou, P dans le composé de formule (II), respectivement, pour donner le composé de formule (I).

**2.** Procédé suivant la revendication 1, où P dans le composé de formule (II) est un radical isocyanate et Q dans le composé de formule (III) est un radical amino.

**3.** Procédé suivant la revendication 1 ou 2, où le composé de formule (I) ainsi obtenu est converti en un sel ou solvate correspondant.

**4.** Procédé pour préparer une composition pharmaceutique, qui comprend :
(a) la préparation du composé de formule (I) ou d'un sel ou solvate physiologiquement acceptable de celui-ci par un procédé suivant l'une quelconque des revendications 1 à 3, et
(b) le mélange du produit du stade (a) avec un excipient ou diluant pharmaceutiquement acceptable et facultativement un ou plusieurs autres agents pharmacologiquement actifs.

**5.** Procédé suivant la revendication 4, qui comprend un stade supplémentaire (c) au cours duquel le mélange obtenu au stade (b) est façonné en un comprimé ou une capsule.